# EUROPÄISCHE PATENTANMELDUNG

(11) **EP 1 666 088 A1**
(43) Veröffentlichungstag der Anmeldung: **07.06.2006**
(21) Anmeldenummer: 06001552.6
(22) Anmeldetag: 20.02.1998
(51) Int. Cl.: A61N 1/36, A61F 9/08, H04N 5/232

(54) **Lernfähiger sensomotorischer Encoder für Seh- oder Hörprothesen**

(30) Priorität: 21.02.1997 DE 19707046
(62) Teilanmeldung aus: 98912345.0
(71) Anmelder: Intelligent Acquisition, LLC, Brookfield, WI 53045-5111 (US)
(72) Erfinder: Eckmiller, Rolf, Prof. Dr., 41460 Neuss (DE)
(74) Vertreter: Graf von Stosch, Andreas

(57) **Zusammenfassung**

Die Erfindung betrifft einen lernfähigen, sensomotorischen Encoder für eine Sehprothese oder Hörprothese, mit einer zentralen Kontrolleinheit für Signalverarbeitungsfunktionen, Überwachungsfunktionen, Steuerfunktionen und externen Eingriffsfunktionen, sowie mit einer Gruppe von adaptiven spatiotemporalen Filtern zur Umsetzung von Sensorsignalen in Stimulationspulsfolgen, wobei eine bidirektionale Schnittstelle zur Kopplung des Encoders mit einer implantierbaren Mikrostruktur 2 zur Stimulation von Nerven- oder Gliagewebe einerseits sowie zur Funktionsüberwachung von Hirnfunktionen andererseits vorgesehen ist.

## Beschreibung

Die vorliegende Erfindung betrifft ein Informationsverarbeitungssystem als lernfähiger, sensomotorischer Encoder für eine Sehprothese oder Hörprothese zur bidirektionalen Kopplung mit implantierten Mikrokontakten zur Stimulation von Nerven-oder Gliagewebe einerseits sowie zur Funktionsüberwachung von Hirnfunktionen andererseits.

Es sind mehrere Versuche bekannt, Sehprothesen für verschiedene Gruppen von Blinden durch Implantation von Mikrokontakten an der Ausgangsschicht der Retina (RET) oder innerhalb des visuellen Cortex (VCO) zu entwickeln und durch Kopplung dieser Implantate mit externen Signalgebern (Encoder) nützliche visuelle Wahrnehmungen auszulösen. Encoder für implantierbare Sehprothesen werden zum Beispiel in US 5,498,521, US 5,351,314 oder WO95/06288 beschrieben, implantierbare Mikrokontakte für Retina, visuellen Cortex bzw. auditorische Systeme sind in US 5,597,381, US 5,569,307, US 5,549,658, US 5,545,219, US 5,496,369, US 5,411,540, US 5,215,088, US 5,109,844 und US 4,628,933 beschrieben. Mit neuronalen Netzen und visuellen Systemen befassen sich die US 5,227,886, EP . 0435559, US 3,766,311 sowie mit der Ansteuerung von Mikrokontakten US 5,571,148, US 5,512,906, US 5,501,703, US 5,441,532, US 5,411,540.

Die Zielgruppen von RET-Projekten leiden unter retinalen Degenerationserkrankungen (z.B. Retinitis pigmentosa; Makula Degeneration), wobei die Schicht der Photorezeptoren degeneriert ist, jedoch zumindest ein Teil der retinalen Ganglienzellen und des dort beginnenden Sehnervs sowie das zentrale Sehsystem noch funktionstüchtig sind. Wie die vorgenannten Druckschriften zeigen, wird an der Entwicklung verschiedener Arten von implantierbaren Mikrokontaktstrukturen (Stimulator), die sich innerhalb des Augapfels an die Ganglienzellschicht der Retina anschmiegen und an der Entwicklung von drahtlosen Signal- und Energieübertragungssystemen zur Verbindung zwischen dem externen Encoder und dem implantierten Stimulator, oder allgemein dem Interface gearbeitet.

Der Erfinder hat sich die Aufgabe gestellt, einen lernfähigen Encoder für eine an der Retina oder der Sehrinde angekoppelte Sehprothese zur Umsetzung von Bildmustern, oder für eine an geeigneten Bereichen des neuronalen Hörsystems angekoppelte Hörprothese zur Umsetzung von Schallmustern in Stimulationssignale durch adaptive, spatiotemporale Filter mit rezeptiven Feldeigenschaften der entsprechenden kontaktierten sensorischen Neurone (RF-Filter) und deren optimaler Einstellung durch neuronale Netze als lernfähige Funktionsapproximatoren weiterzuentwickeln.

Die Zielgruppen von VCO-Projekten haben typischerweise keine verwertbaren Sehnervfunktionen mehr und erfordern daher eine Implantation von z.B. kammähnlichen Mikrokontaktstrukturen in Regionen des visuellen Cortex, also der Sehrinde, die direkt dem Schädeldach zugewandt sind.

Es sind auch mehrere Arten von Hörprothesen (z.B. Cochlear-Implant) mit implantierten Mikrokontakten bekannt geworden, die Tauben eine teilweise Wiedergewinnung von Hörwahrnehmungen ermöglichen.

Die gegenwärtig konzipierten Implantatsysteme sehen keine Informationsverarbeitung der dynamischen Bildmusterdaten innerhalb des Sehsystems (z.B. Retina), jeweils vom optischen Eingang bis zu der durch implantierte Mikrokontakte kontaktierten neuronalen Schicht (z.B. Ganglienzellschicht der Retina, oder Neuronenschicht im visuellen Cortex) vor. Statt dessen werden einfache Bildmuster (z.B. Linie) direkt als Stimulationssignale an die örtlich verteilten Mikrokontakte ohne individuell angepaßte Informationsverarbeitung als Ersatz für den hier technisch zu überbrückenden Teil des Sehsystems (z.B. Retina) weitergeleitet. Dem so rudimentär und nicht angepaßt erregten Sehsystem fällt dabei die kaum lösbare Aufgabe zu, aus den örtlich und zeitlich nicht korrekt vorverarbeiteten oder codierten Signalverläufen Sehwahrnehmungen zu generieren, die dem Bildmuster hinreichend ähnlich sind. Ferner wird die physiologische Verstellbarkeit der Sehempfindlichkeit (Leuchtdichte-Adaptation) über etwa 10 Dekaden und die damit verbundene funktionelle Änderung der rezeptiven Feldeigenschaften in technischen Photosensorsystemen nicht berücksichtigt.

Die gegenwärtig entwickelten Implantatsysteme nutzen die technischen Möglichkeiten zum Einsatz der Sehprothese nicht zur Warnung vor Gefahren und Meldung technisch erkannter Muster an den Implantatträger. Entsprechendes gilt für gegenwärtig entwickelte Implantatsysteme für das Hörsystem.

Das Sehsystem erwartet aufgrund seiner während der Ontogenesefestgelegten und durch langjährige Sehwahrnehmungserfahrungen vor Beginn der Blindheit stabilisierten Struktur und Funktion z.B. von jeder retinalen Ganglienzelle über den Sehnerv eine bestimmte Art der Informationsverarbeitung der Bildmusterdaten. Diese durch die zugehörigen rezeptiven Feldeigenschaften (RF) des Neurons neurophysiologisch umschriebene und für jedes Neuron sehr unterschiedliche Erwartung wird nicht erfüllt, sofern z.B. die Funktion der elektronisch mit starrer Vorverarbeitung simulierten Retina nicht für jeden einzelnen, durch Implantation hergestellten, Stimulationskontakt individuell angepaßt werden kann. Entsprechendes gilt für das Hörsystem.

Das für den normalen Sehvorgang erforderliche Zusammenwirken des von der Retina gespeisten, zentralen visuellen Systems mit einem Augenbewegungssystem und einem Akkommodationssystem wird als sensomotorisches 'Active Vision' System bezeichnet. Derartige Systeme für Mustererkennung, Objektverfolgung, Positionserfassung von Objekten, usw. werden in bisherigen Ansätzen nicht berücksichtigt. Das Sehsystem benötigt jedoch Augenbewegungen für alle Sehleistungen und erbringt nicht nur Erkennungsleistungen (was?), sondern vor allem auch Positionserfassungsleistungen (wo?) zur Orientierung im Raum, die für Blinde im Bemühen um die teilweise Wiedergewinnung des Sehvermögens oberste Priorität haben. Die Auslösung von visuell induzierten Augenbewegungen ist jedoch bei der durch Implantatsysteme realistischen Stimulation von nur einem kleinen Bruchteil der retinalen Ganglienzellen, oder der Neuronen im visuellen Cortex nicht zu erwarten. Daher kann das Sehsystem mit den bisher in Entwicklung befindlichen Implantaten die auf dem normalen Zusammenwirken mit Augenbewegungen basierenden Sehwahrnehmungen wenn überhaupt, nur sehr schlecht leisten.

Bei verschiedenen Blinden treten außerdem unerwünschte, nicht visuell induzierte Augenbewegungen mit langsamen und schnellen Phasen auf, die den optimalen Einsatz, also auch die Akzeptanz dieser Art von Sehprothesen erheblich behindern können. Wird z.B. der Encoder mit Photosensorarray am Augapfel befestigt, so wird der gewünschte Fixationsort laufend durch die unerwünschten Augenbewegungen verstellt. Wird andererseits der Encoder z.B. in ein Brillengestell integriert, so kann das Sehsystem die Bildmuster mit den nicht darauf abgestimmten Augenbewegungen als mißverständliche Sehwahrnehmungen z.B. mit Scheinbewegungen auswerten, wie dies z.B. bei Schwindelwahrnehmungen der Fall ist.

Ohne die Möglichkeit von visuell induzierten, realen Augenbewegungen und andererseits zusätzlich im Konflikt mit unerwünschten spontanen Augenbewegungen, ist die visuelle Orientierung im Raum, die Positionserfassung diverser Objekte relativ zur Lage des eigenen Körpers, z.B. für das angestrebte Ergreifen eines Türgriffes mit bisher in Entwicklung befindlichen Sehprothesen, die zur Änderung der Blickrichtung auf Kopf-Rumpfbewegungen angewiesen sind, nur schwer möglich. Die zu implantierenden Strukturen haben eine sehr begrenzte Zahl von Mikrokontakten. Die Zahl der effektiv verwendbaren Mikrokontakte ist noch kleiner, da die Kontakte durch die Implantation nur zum Teil hinreichend günstig relativ zu einer Nervenzelle oder Faser positioniert werden können, daß durch Verwendung einzelner Kontakte, oder Kontaktpaare Nervenaktionspotentiale mit vertretbar geringen Stimulationsamplituden ausgelöst werden können. Bei den bisherigen Entwicklungen gibt es kaum Möglichkeiten, die Zahl der dauerhaft und selektiv kontaktierten Neuronen über die bei Implantation zufällig geschaffene Zahl hinaus zu erhöhen. Dies ist ein Grund für die nur geringe zu erwartende visuelle Wahrnehmungsqualität. Entsprechendes gilt für Implantatsysteme des Hörsystems.

Die für eine Implantation gegenwärtig entwickelten Mikrokontaktstrukturen sowie Signal- und Energieübertragungssysteme für Sehprothesen funktionieren unidirektional vom externen Encoder zum implantierten Stimulator und bieten daher keine Möglichkeit zur laufenden Überwachung der neuronalen Impulsaktivität der stimulierten Neuronen. Dadurch kann die Stimulationspulssequenz nicht an die Spontanaktivität der Neuronen angepaßt werden. Ferner kann die Auslösung von neurobiologischen Impulsen durch Stimulationspulse nicht direkt überwacht werden. Außerdem fehlt eine sichere Impulsüberwachungsmöglichkeit für eine mögliche zeitliche Abstimmung und Synchronisation der Impulsfolgen mehrerer Neuronen. Entsprechendes gilt für Implantatsysteme des Hörsystems.

Es ist die Aufgabe der vorliegenden Erfindung, diese Nachteile zu beseitigen und einen lernfähigen, sensomotorischen Encoder zu schaffen.

Diese Aufgabe wird von einer Vorrichtung mit den Merkmalen des Anspruchs 1 gelöst.

Weil der Encoder in bidirektionaler Kopplung mit implantierten Mikrokontakten an der Retina, oder dem visuellen Cortex nach einer - vorzugsweise mit Hilfe neuronaler Netze im Dialog mit dem Implantatträger durchgeführten - Funktionsabstimmung auf den individuellen Bedarf arbeitet, kann durch technische Bildmusterverschiebungen und simulierte Augenbewegungen diverse visuelle Erkennungs-, Verfolgungs- und Positionserfassungsaufgaben sowie die Meldung von Gefahren und technisch erkannten Mustern geleistet werden, die Zahl der selektiv erreichbaren Stimulationsorte funktionell erhöht sowie die neuronale Aktivität einzelner zu stimulierender Neuronen überwacht werden. Ferner kann der hier offenbarte Encoder Funktionen der Leuchtdichteadaptation und der Zusammenstellung eines visuellen Arbeitsbereiches aus Ausschnitten eines großen Funktionsbereiches leisten.

Bei Einsatz für eine Hörprothese wird der lernfähige Encoder entsprechende Leistungen im auditorischen Bereich erbringen.

Bei einer bevorzugten Ausführungsform des Encoders für eine Sehprothese ist ein digitaler Signalprozessor (DSP), beispielsweise das Modell C80 von Texas Instruments, mit einem Photosensorarray mit Optik als Lichtmusterempfänger, einem Vorverabeitungsmodul für visuelle Muster, einem Pulssignalsender und Empfänger zur bidirektionalen Kommunikation mit der implantierten Struktur, mehreren Signalinterfaces zur Kommunikation mit der Bewertungseingabeeinheit, dem Kopfbewegungssensor, dem Augenbewegungssensor, dem Wahrnehmungs-, Warn- und Erkennungssystem, Muster- und Objektmeldesystem und dem externen Überwachungs- und Steuersystem in einem Brillengestell integriert . Die diversen lernfähigen Informationsverarbeitungsfunktionen insbesondere für RF-Filter, Dialogmodul, Mustererkennung und Active Vision Funktionen sind im DSP mit einer zentralen Kontrolleinheit realisiert. Der Benutzer empfängt einerseits Signale als Stimulationspulse oder Sinneswahrnehmungen vom Encoder empfängt und sendet andererseits Signale über Kopf- und Augenbewegungen, sowie die Bewertungseingabe und neuronale Aktivität an den Encoder. Der Encoder kann aufgrund einer bidirektionalen drahtlosen Signal-und Energieübertragung in einem Brillengestell, einer Haftschale am Auge, am Körper, oder an einem körperfernen Ort befestigt werden. Schließlich umfaßt der spatiotemporale Funktionsraum der RF-Filter z.B. für den Einsatz als Retina Encoder die rezeptiven Feldeigenschaften retinaler Ganglienzellen oder anderer intraretinaler Neuronenklassen der Primatenretina. Entsprechendes gilt für vorteilhafte Ausgestaltungen des Encoders bei Kopplung mit Neuronen der Sehrinde, oder im Fall einer Hörprothese für die Kopplung mit Neuronen des Hörsystems.

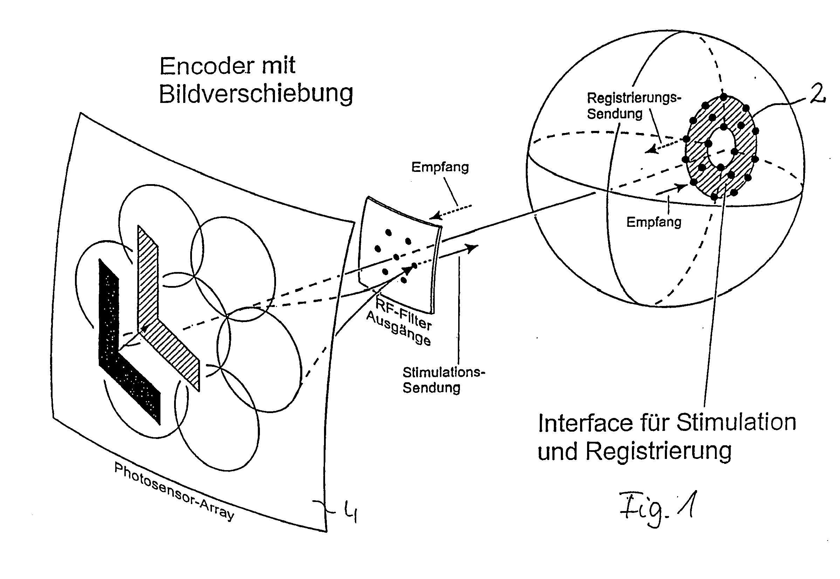

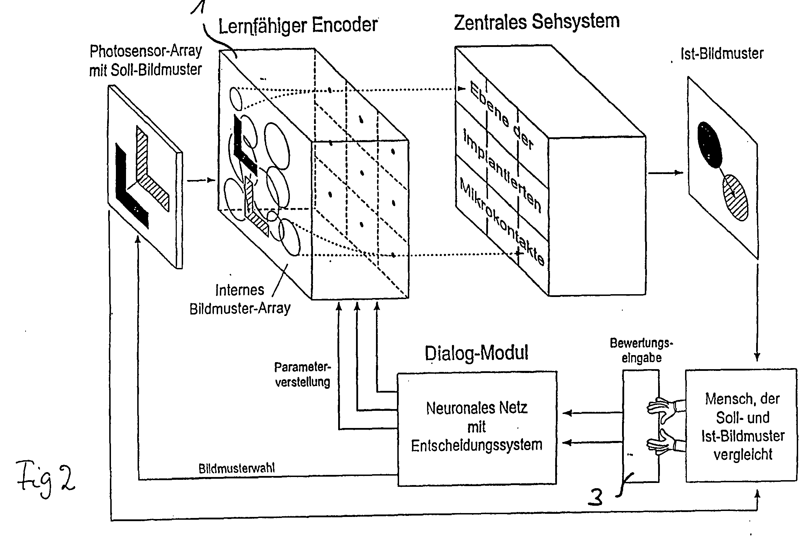

Eine vorteilhafte Ausgestaltung eines Verfahrens zur Einstellung der RF-Filter des Encoders im Dialog mit dem Benutzer ist durch Fig. 1 und Fig. 2 schematisch dargestellt. Die RF-Filter sind als spatiotemporale Filter realisiert, deren spatiale und temporale Funktionsparameter in einem zur Annäherung der rezeptiven Feldeigenschaften visueller Neuronen hinreichend großen Funktionsraum modifiziert werden, nämlich durch an geeigneten Stellen in den Filteralgorithmen plazierte, extern zugängliche Parameteraustauschpunkte. Ein Mensch teilt als normalsichtige Person oder Implantatträger in einem wahrnehmungsbasierten Dialog mit dem Encoder den Wahrnehmungsvergleich zwischen Soll- und Ist-Muster, z.B. durch eine als Zeile von mehreren Schiebereglern gestaltete Bewertungseingabeeinheit (s. Fig. 2) an ein technisches neuronales Netz mit nichtüberwachter Lernregel mit, und das neuronale Netz legt den nächsten Parametervektor für das RF-Filter sowie das nächste Soll-Muster mit dem Ziel der Verringerung der wahrgenommenen Musterdifferenz im nächsten Dialogschritt fest. Bei der Suche nach optimalen Parametervektoren für die RF-Filter können in dem Dialogmodul von einem neuronalen Netz mit nichtüberwachtem Lernen jeweils Parametervektoren erzeugt werden, die für ein gegebenes präsentiertes Lichtmuster zu einer bestimmten Sehwahrnehmung führen und entsprechend subjektiv bewertet werden. Alternativ kann in dem Dialogmodul ein anderes Parameter-Einstellungssystem Sequenzen von Parametervektoren zur virtuellen Bewegung im Funktionsraum des RF-Filters z.B. als kontinuierliche Trajektorien nach Art von Scanning oder Sweepverläufen als regellose Folgen oder als Folgen von neurophysiologisch besonders typischen Filterfunktionen erzeugen,und der Benutzer während dieser in einem geeigneten Zeittakt ablaufenden Sequenz gelegentlich aus dem Zusammenwirken des gegebenen Lichtmusters, des nachgeschalteten Vorverarbeitungsmoduls, des nachgeschalteten RF-Filters und dem über den zugehörigen Mikrokontakt angekoppelten Teil des zentralen Sehsystems verursachte "sinnvolle" Wahrnehmungen melden. Anschließend wird in dem so gefundenen Bereich des Filterfunktionsraumes eine genauere Parameteroptimierung wahrnehmungsbasiert durchgeführt. Eine vorteilhafte Ausgestaltung der dialogbasierten Einstellung der RF-Filter des Encoders einer Hörprothese erfolgt entsprechend.

Bei der Erzeugung asynchroner Impulsfolgen werden die Ausgangssignale der einzelnen RF-Filter durch geeignete Umsetzer-Algorithmen von den quasikontinuierlichen Zeitfunktionen der RF-Filter in asynchrone Impulsfolgen entsprechend der Aktivität visueller Neuronen im Primatensehsystem gewandelt und Impulsfolgen-Zeitverläufe sowie Zeitpunkte des Auftretens einzelner Impulse durch variable Zeitverzögerungsglieder in der Dialogphase verstellt.

Normalsichtige Personen können einen Wahrnehmungsvergleich zwischen einem Soll-Muster auf dem linken Schirm und einem korrespondierenden Ist-Muster auf dem rechten Schirm durchführen. Eine vorteilhafte Ausgestaltung des Sehsystemmodelles für den Wahrnehmungsvergleich bei normalsichtigen Personen besteht darin, daß es für eine Reihe von RF-Filtern, einzeln oder als Gruppe die jeweils zugehörige inverse Abbildung leistet und dadurch in Hintereinanderschaltung mit dem Encoder jeweils für genau festgelegte Parametervektoren auf dem rechten Schirm ein Ist-Muster mit großer Ähnlichkeit zu dem Soll-Muster erzeugt. Zu Beginn des Dialogs sind die Parametervektoren der RF-Filter auf beliebige Anfangswerte gestellt, so daß zunächst ein deutlicher Unterschied zwischen den Mustern besteht, der jedoch im Verlauf des Dialogs mit nichtüberwachtem Lernen laufend geringer wird.

Die Funktionsanpassung der RF-Filter für Implantatträger im wahrnehmungsbasierten Dialog erfolgt im Vergleich zur Funktionsanpassung für normalsichtige Personen dadurch, daß die Ist-Wahrnehmung nicht auf einem Monitor, sondern nur dem Implantatträger intern zugänglich ist und daß die Soll-Wahrnehmung dem Implantatträger z.B. als Sprachinformation oder als taktiles Berührungsmuster auf der Haut vermittelt wird. Beim Einsatz in Hörprothesen ergibt sich Entsprechendes, wobei statt des nicht zugänglichen Hörorgans z.B. neben dem Tastsinn der hier verfügbare Gesichtssinn zur Übermittlung der Soll-Wahrnehmung verwendet werden kann.

Die zeitliche Kopplung der von mehreren RF-Filters des Encoders zur Auslösung von Nervenzellimpulsen erzeugten asynchronen Impulsfolgen besteht darin, daß die Sendezeitpunkte der einzelnen Impulssignale durch steuerbare Zeitverzögerungsglieder so variiert werden, daß sich eine zeitliche Kopplung bis hin zum genau synchronen Auftreten ergibt, daß die Variation der Zeitverzögerung vom Implantatträger gesteuert wird, im Dialog wahrnehmungsbasiert über ein neuronales Netz erfolgt oder extern gesteuert wird, daß die Wahl der zeitlich zu koppelnden Impulsgruppen sowohl vom RF-Filter kommende als auch im Interface registrierte Impulse berücksichtigen kann und daß angesichts der sehr unterschiedlichen momentanen Impulsraten der verschiedenen RF-Filter geeignete Kriterien für die Beteiligung einzelner Impulse an zu koppelnden Impulsgruppen festgelegt werden.

Zur funktionellen Erhöhung der Zahl und Trennschärfe der selektiv erreichbaren Stimulationsorte bei einer gegebenen Zahl von stationär implantierten Mikrokontakten werden die Impulssignale von einem gegebenen RF-Filter an mehrere, örtlich benachbarte Mikrokontakte geleitet. Die aufgrund der Encoderbefehle genau für jeden Mikrokontakt festgelegten und entsprechend im Interface bezüglich Stromamplitude, Polarität und Phasenlage decodierten Stimulationszeitfunktionen charakteristische Zeitverläufe des elektromagnetischen Feldes im Bereich der zu stimulierenden Neuronen bewirken, daß diese durch Superposition aufeinander abgestimmter Stimulationssignale an mehreren Mikrokontakten bewirkten Feldverteilungen örtlich und zeitlich selektiv neuronale Impulserregungen auslösen, daß die selektiven Stimulationsorte durch geeignete Variation der superponierten Stimulationssignale schnell gewechselt werden können und daß die entsprechende Variation diversen Parameter der aufeinander abgestimmten Stimulationssignale im wahrnehmungsbasierten Dialog mit dem Implantatträger über ein neuronales Netz, oder andere Signalvariationsverfahren wie z. B. eine kontinuierliche, sweepartige automatische Verstellung der Funktionsparameter der in Überlagerung auf das Nervengewebe einwirkenden Stimulationsimpulse an benachbarten Elektroden zur Bestimmung möglichst vieler, zu selektiver und trennscharfer neuronaler Erregung führender, Stimulationsorte erfolgt. Ferner wird durch Vergleich registrierter neuronaler Impulse mit den Stimulationssignalen die Optimierung der Stimulationszeitfunktionen bezüglich angestrebter Einzelzellselektivität und dauerhafter Bioverträglichkeit verbessert.

Zur Simulation von Augenbewegungen zum Einsatz des Encoders in einer Sehprothese wird eine Bildmusterverschiebung elektronisch in der Eingangsstufe des Encoders durch optische Variation der Blickrichtung z.B. mit Hilfe bewegter Spiegel oder durch Bewegung der Photosensoren zur Simulation von Augenbewegungen realisiert. Kopfbewegungen und Augenbewegungen werden durch mehrdimensional funktionierende, mikrominiaturisierte Bewegungsdetektoren detektiert und eine neuronale oder konventionelle Bewegungsregelung bzw. Steuerung wird unter Verwendung der detektierten Kopf- und Augenbewegungssignale und der Bildmusterverschiebung realisiert. Schnelle und langsame Augenbewegungen für Aufgaben der Mustererkennung, schnellem Umherblicken oder Augenfolgebewegungen werden erzeugt, und durch geeignete Augenbewegungssequenzen mit schnellen und langsamen Phasen erfolgt eine optimale Anpassung des sensorischen Datenstromes an die Aufnahmebereitschaft des zentralen Sehsystems. Bezüglich der Erzeugung von Augenfolgebewegungen besteht eine vorteilhafte Ausgestaltung darin, daß ein lernfähiger, neuronaler Prädiktor die zunächst unbekannte Bewegungszeitfunktion des zu verfolgenden Objektes aus dem Positions- und Bewegungsfehler seiner Projektion auf dem Photosensor-Array erfaßt, mit Hilfe geeigneter nichtlinearer, lernfähiger Prädiktions-Algorithmen unter Berücksichtigung der analysierten Frequenzanteile der Objektbewegung intern mit rasch zunehmender Gewißheit eine Objektverfolgungs-Zeitfunktion mit minimaler Verzögerung bzw. sogar mit geringer Voreilung generiert und bei vorübergehendem Verschwinden des verfolgten Objektes z.B. ähnlich wie bei Augenfolgebewegungssystem von Primaten eine Bewegungszeitfunktion erzeugt, die je nach Art des Objektes zu einer Fortsetzung der Verfolgung bei Wiederauftauchen des relativ zum Sensorarray bewegten Objektes mit minimalem Positions- und Bewegungsfehler führt.

Zur Detektion von Augen- und Kopfbewegungen und zur Kompensation unerwünschter Augenbewegungen werden unter Verwendung der detektierten Kopf- und Augenbewegungssignale und der simulierten Augenbewegungen mit Hilfe einer neuronalen oderkonventionellen Bewegungsregelung bzw. Steuerung schnelle und langsame Augenbewegungen erzeugt. Mit Hilfe des Regelkreises werden nach einer entsprechenden Lernperiode unerwünschte Augenbewegungen kompensiert und daß ein aus Kopfbewegungsdetektor, Bildmusterverschiebung und einem neuronalen Netz bestehender, in einer Lernphase optimierter Regelkreis eine hinreichend gute Simulation des vestibulookulären Reflexes (also der automatisch reflexartigen Stabilisierung der Blickrichtung im Raum durch Augenbewegungen, die den auftretenden Kopfbewegungen genau entgegenwirken) leistet die Lagestabilisierung der Bildmuster bei natürlichen Kopf-Rumpfbewegungen durch entsprechend kompensatorische Augenbewegungen.

Simulierte Augenbewegungen sowie kompensatorische Augenbewegungen sind als separat wählbare Programme verfügbar. Die einzelnen Bewegungs- und Verstellungsmoden können als separate, oder kombinierte Programme vom Implantatträger gewählt, einem automatischen Betrieb zugewiesen oder extern festgelegt werden.

Der Implantatträger kann durch eine mitgeführte Befehlseingabeeinheit, z.B. als Manipulandum mit Tastatur, die gewünschten 'Active Vision' Funktionen, wie etwa Umherblicken, oder Objektverfolgung anwählen.

Die laufende Übermittlung der aktuellen Position von visuell oder auditorisch erfaßten Objekten im Raum an den Implantatträger besteht darin, daß die vom Encoder unter Auswertung von Bildmuster-, Schallmuster-, Augen- und Kopfbewegungen ermittelte Objektposition mit Hilfe eines geeigneten, mitgeführten Signalgebers an ein geeignetes Sinnesorgan, z.B. den Tastsinn, gemeldet wird und daß der Encoder durch interne Mustererkennungsprogramme, insbesondere in Verbindung mit automatisch ablaufenden Augenbewegungen, den Implantat-Träger vor Hindernissen bzw. Gefahren warnt sowie Art und Position technisch erkannter Muster oder Objekte meldet.

Ein Überwachungssystem für eine teilweise sensomotorisch autonom agierende implantierte Struktur des Encoders besteht darin, daß die implantierten Mikrokontakte sowohl zur Stimulation, als auch zur Registrierung neuronaler Impulse verwendet, daß die registrierten Impulse und weiteren physikalischen oder chemischen Signale von der implantierten Struktur durch geeignete Vorverstärker und optische oder elektromagnetische Sender an den Encoder gemeldet und daß dort die registrierten neuronalen Signale für verschiedene Zwecke der Encoderfunktion weiterverarbeitet werden.

Zur technischen Anpassung des Leuchtdichte-Arbeitsbereiches aus einem z.B. über sechs bis zehn Leuchtdichtedekaden reichenden Funktionsbereich des Photosensorarrays, der sowohl große Teile des skotopischen Bereiches der Dunkeladaptation als auch des photopischen Bereiches der Helladapation umfaßt, wird eine schnell verstellbare elektronische Abbildung auf einen internen Arbeitsbereich für den Encoder bezüglich Größe und Adaptationsleuchtdichte wahrnehmungsbasiert, automatisch gewählt oder vom Benutzer eingestellt. Dadurch werden visuelle Szenen von sehr unterschiedlicher Leuchtdichte in den jeweiligen Arbeitsbereich komprimiert und Kontrastoptimierung sowie Blendungsvermeidung erzielt. Ferner besteht eine vorteilhafte Ausgestaltung darin, daß die RF-Filterfunktionen z.B. entsprechend neurobiologisch bekannten Verläufen mit dem Adaptationsbereich verstellt werden können.

Bei einer vorteilhaften Ausführungsform ist ein Vorverarbeitungsmodul vorgesehen, das zur Erkennung charakteristischer Muster und/oder zur Datenreduktion eingerichtet ist. Dabei kann das Vorverarbeitungsmodul helle und dunkle Bildbereiche unterschiedlicher Beleuchtung erkennen, voneinander abgrenzen und zu einem Gesamtbild mit jeweils regional optimalem Kontrast integrieren, ebenso können nahe und entfernt liegende Bildbereiche voneinander getrennt bezüglich Scharfeinstellung optimiert und danach wieder als Gesamtbild integriert werden, und schließlich können charakteristische Muster wie z. B. Warnschilder hervorgehoben werden. Diese Vorverarbeitung kann zur Verbesserung der Bilddarstellung, aber auch zur Erzeugung von Warnhinweisen sowie zur Datenreduktion im Kommunikationskanal zwischen der Kamera und dem Encoder dienen.

Weiter ist es von Vorteil, wenn in einem Verfahrensablauf die Adaptation der Akkommodation zunächst, beispielsweise im Vordergrund, scharf eingestellt werden, diese Bereiche gespeichert werden und dann zweite Bereiche des Blickfeldes als Bild scharf eingestellt werden, beispielsweise der Hintergrund eines Blickfeldes. Die dabei unscharf werdenden ersten Bereiche werden aus dem zweiten Bildmuster ausgeblendet und durch die ersten gespeicherten, scharfen Bereiche ersetzt. Hierdurch wird eine im Rahmen der geometrischen Optik nicht erreichbare Tiefenschärfe erzeugt. Bei sich zyklisch wiederholendem Ablauf dieser Verfahrensschritte und einem entsprechenden Bildausgleich kann dieser Vorgang von einem Benutzer unbemerkt bleiben, so daß auch bei geringen Lichtstärken und langen Brennweiten der zugehörigen Optik ein Bild von großer Tiefenschärfe scheinbar erreicht wird.

Das lernfähige Vorverarbeitungsmodul verarbeitet das Bildmuster oder Schallmuster durch neuronale Netze oder in Hardware verfügbare Vorverarbeitungs-Algorithmen für eine Sehprothese insbesondere bezüglich Farbe, Kontrast, Kantendetektion, Segmentierung und Figur-Hintergrund Trennung oder für eine Hörprothese z. B. bezüglich Unterdrückung von Störgeräuschen, unerwünschten Formanten und Separation einzelner Schallquellen, derart, daß das nachgeschaltete RF-Filter Array erheblich vereinfachte und teilweise aus dem Musterraum in einen Merkmalsraum abgebildete Bildmuster oder Schallmuster erhält, die dem kontaktierten Teil des Sehsystems bzw. Hörsystems möglichst gut angepaßt sind. Die verschiedenen Vorverarbeitungsfunktionen werden vom Benutzer direkt oder durch wahrnehmungsbasierten Dialog eingestellt bzw. gewählt oder automatisch eingestellt. Die Übermittlung eines derart vereinfachten Bildes an den Retinaencoder ist für die Gestalterkennung auch bei einer beschränkten Anzahl von kontaktierten Nervenzellen des Sehsystems vorteilhaft. Bei der Vorverarbeitung für Hörprothesen ergeben sich entsprechenden Vorteile, wenn komplexe Schallmuster im Wege der Vorverarbeitung für die Spracherkennung aufbereitet werden.

Im folgenden wird eine Ausführungsform der erfindungsgemäßen Encodervorrichtung und des zugehörigen Verfahrens anhand der Zeichnung erläutert. Es zeigen:
- Fig.1:: eine Sehprothese am Beispiel eines Retina Implants;
- Fig.2:: einen lernfähigen Encoder mit Dialogsystem; sowie
- Fig.3:: Schema der Detektoren von Kopfbewegungen und Augenbewegungen, des Retinaencoders vor einem Auge sowie einer Halsmanschette zur taktilen Rückmeldung von Objektpositionen;
- Fig. 4:: eine schematische Darstellung einer implantierten Mikrokontaktstruktur zur Stimulation von nicht unmittelbar kontaktiertem Nervengewebe;
- Fig. 5:: die Darstellung einer Szene mit sehr unterschiedlichen Helligkeitsbereichen und Bereichen unterschiedlicher Fokussierungsentfernung; sowie
- Fig. 6:: den Verlauf der Helligkeitsstufen in der Kameraabbildung für vier verschiedene Bildbereiche aus Figur 5.

Fig. 1 zeigt schematisch eine Sehprothese am Beispiel eines Retina Implants mit einem in einem Brillengestell befestigten, lernfähigen, sensomotorischen Encoder mit Bildverschiebung, einem nahe der Ganglienzellschicht im Auge implantierten Interface für Stimulation und Registrierung als Mikrokontaktstruktur 2 mit Zusatzelektronik und einer bidirektionalen, drahtlosen Signal- und Energieübertragung zwischen Encoder und Interface. Die einzelnen spatiotemporalen Filter (RF-Filter) des Encoders mit jeweils etwa runden Ausschnitten des Photosensorarrays 4 in der Eingangsebene (links) und zugehörigen Signalausgängen in der Ausgangsebene (Mitte) repräsentieren typische rezeptive Feldeigenschaften z.B. von Ganglienzellen der Primatenretina, oder von Neuronen im visuellen Cortex und sind durch einen Parametervektor individuell funktionell verstellbar. Das Interface empfängt nicht nur Stimulationssignale vom Encoder, sondern sendet seinerseits auch registrierte neuronale Impulssignale an den Encoder. Die zusätzlich zum lernfähigen Encoder gehörenden Komponenten, wie z.B. die zentrale Kontrolleinheit, oder das den RF-Filtern vorgeschaltete Vorverarbeitungsmodul sind in Fig. 1 nicht dargestellt.

Fig. 2 zeigt das Schema eines Encoders 1 mit Dialogsystem in Kopplung mit dem zentralen Sehsystem, entweder als Modell (Einsatz bei normalsichtiger Person), oder als reales System von der Ebene der implantierten Mikrokontakte bis zur visuellen Wahrnehmung (Einsatz bei Implantatträger). Der Winkel als exemplarisches Bildmuster auf dem Photosensorarray (links) repräsentiert gleichzeitig ein Sollmuster auf einem Monitor, welches sich nach rechts oben bewegt Auf dem anschließenden internen Bildmusterarray, welches elektronisch im Encoder realisiert ist, bewegt sich der Winkel in dem Beispiel von einer anderen Ausgangsposition aus in eine andere Richtung, um die Funktion einer technischen Bildverschiebung nach Anspruch 18 anzudeuten. Der Schirm rechts zeigt das zugehörige Ist-Bildmuster und repräsentiert entweder einen zweiten Monitor (normalsichtige Person), oder eine virtuelle Projektion des visuellen Wahrnehmungsraumes (Tmplantatträger). Die sich von links oben nach rechts unten bewegende elliptische Scheibe repräsentiert das korrespondierend wahrgenommene Ist-Bildmuster. Der Mensch (unten rechts) artikuliert seine subjektive Bewertung des Vergleiches zwischen Soll- und Ist-Muster über eine mehrkanalige Bewertungseingabe. Das Dialogmodul als neuronales Netz mit Entscheidungssystem (unten) bildet die Ausgangssignale der Bewertungseingabe auf einen Parametervektor zur Verstellung der RF-Filter Bei Austausch von Bildmustern durch Schallmuster und des Sehsystems durch das Hörsystem gilt Fig. 1 entsprechend für den Einsatz des Encoders für Hörprothesen.

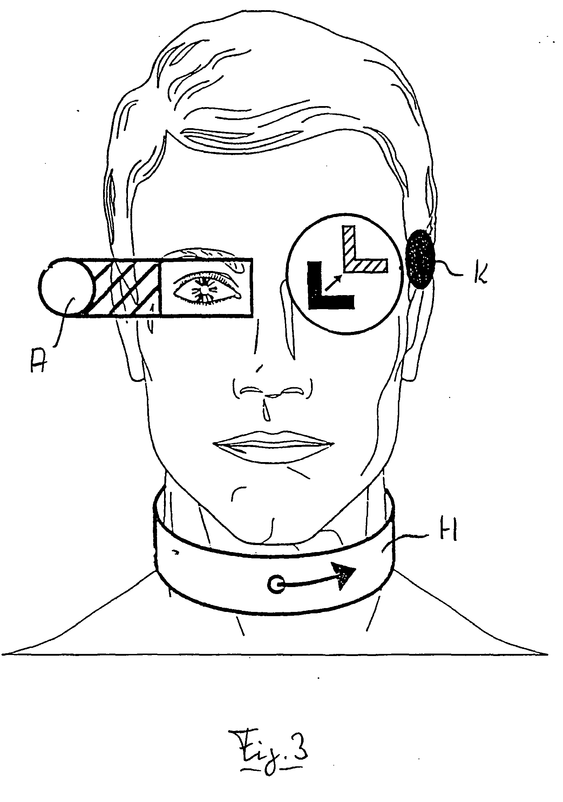

Fig. 3 zeigt schematisch eine vorteilhafte Ausgestaltung des Encoders bezüglich der Positionierung eines mehrdimensional funktionierenden Kopfbewegungssensors K oberhalb des Ohres, eines mehrdimensional funktionierenden Augenbewegungssensors A sowie den in einem Brillengestell integrierten Encoder mit angedeutetem bewegtem Bildmuster. Eine Halsmanschette H zur Erzeugung von lokalen taktilen Empfindungen z.B. durch Vibration gibt dabei dem Benutzer Informationen über die relative Lage eines Objekts zu ihm selbst. Wandert ein vor ihm liegendes Objekt nach links aus dem Blickfeld, so wandert der auf dem Hals erzeugte Vibrationsbereich ebenfalls nach links, wie es in Fig. 3 angedeutet ist.

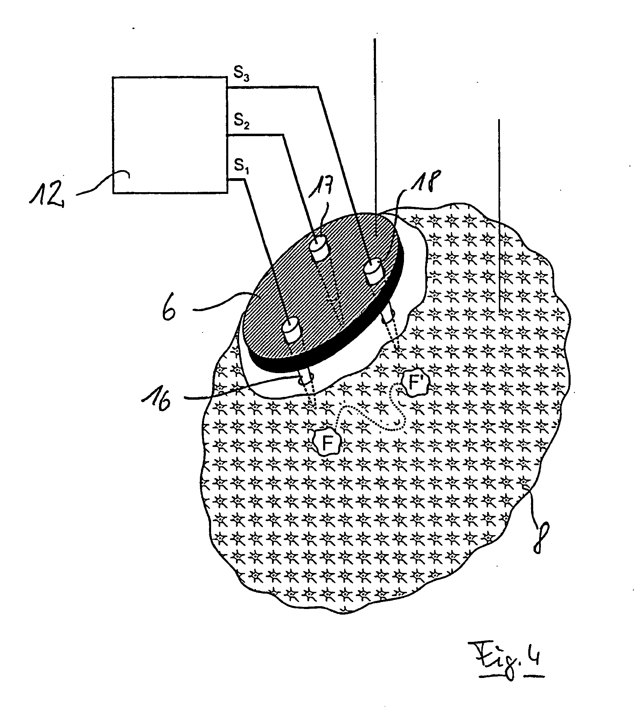

Die Figur 4 zeigt eine veranschaulichte Darstellung von Mikrokontakten 6, die in Nervengewebe 8 eingreifen. Im vorliegenden Beispiel sind drei Mikrokontakte 16, 17, 18 in das Nervengewebe 8 implantiert und dort mehr oder weniger zufällig nahe bestimmten Nervenzellen positioniert. Die Mikrokontaktstruktur 6, 16, 17, 18 ist in jedem Fall wesentlich grober als die Matrix der Nervenzellen 8. Die Mikrokontakte 16, 17, 18 werden über den Stimulator 12 mit Signalen S1, S2 und S3 versorgt.

Um eine gezielte neuronale Anregung zu schaffen, muß beispielsweise ein Stimulationsfokus F erreicht werden, der nicht unmittelbar von einem Mikrokontakt betroffen werden kann. Der Stimulationsfokus F kann aber erreicht werden, indem die Signale S1, S2 und S3 mit unterschiedlichen Stärken, Zeitverläufen und vor allen Dingen zeitlichen Abständen zueinander zu den Elektroden 16, 17 und 18 geleitet werden. Die Überlagerung der erzeugten Signale kann dann so eingerichtet werden, daß das Zusammentreffen der Signale im Bereich des angestrebten Stimulationsfokus F die Reizschwelle einzelner oder weniger Nervenzellen überschreitet, während die Addition der Signalverläufe im übrigen Bereich des Nervengewebes unterhalb der Reizschwelle bleibt.

Durch Änderung der zeitlichen Abfolge und des zeitlichen Signalverlaufs der verschiedenen aufeinander abgestimmten Signale kann auch der Stimulationsfokus von F nach F' verlagert werden. Für den Abgleich dieser Anregungsfunktionen, die einen Stimulationsfokus erreichen, der nicht unmittelbar mit Elektroden in Verbindung steht, ist ein Lernvorgang erforderlich. Da man nicht genau weiß, welcher Stimulationsfokus F, . F' für eine bestimmte neurale Anregung angesprochen werden muß, kann die lernfähige sensomotorische Kontrolleinheit nur ein bestimmtes Signalmuster anbieten, das der Implantatträger - über eine Sinneswahrnehmung oder eine andere Sensordatenauswertung dann beurteilt. Ein zweites Signalmuster, das gegenüber dem ersten verändert wurde, wird dann ebenfalls daraufhin beurteilt, ob es die gezielte neurale Anregung erreicht oder nicht. Der Benutzer braucht lediglich zu sagen, ob das spätere Signalmuster besser oder schlechter als das vorherige geeignet ist. Mit dem Regelmechanismus eines neuronalen Netzes wird im Laufe des Regelvorgangs eine optimale Signal-Zeitfunktion für die Elektroden 16, 17, 18 zur Anregung des Stimulationsfokus F ermittelt.

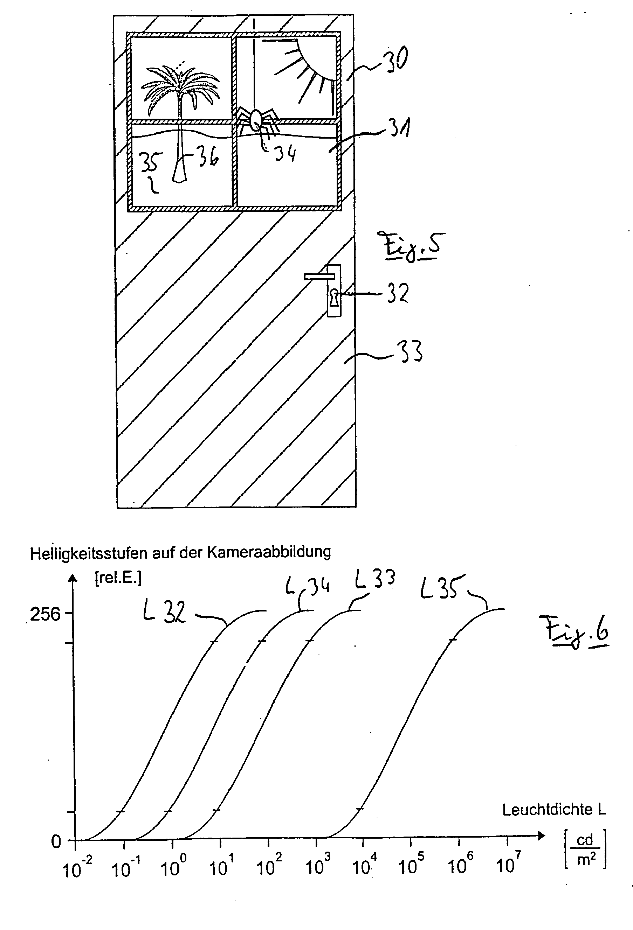

Die Figur 5 zeigt eine durch die Fotosensoren in der Praxis wahrgenommene Szene, bei der eine Terrassentür 30 aus einem Raum heraus betrachtet wird. Die Tür 30 weist ein Fenster 31, ein Schlüsselloch 32 und ein Türblatt 33 auf. Vor der Tür hängt eine Spinne 34, und durch das Fenster 31 ist eine Strandszene 35 sichtbar.

Die Beleuchtungsunterschiede in dieser Szene liegen zwischen etwa 10⁻¹ cd/m² im Bereich des Türschlosses über 10⁰ cd/m² im Bereich der Spinne und 10¹ cd/m² im Bereich des Türblattes bis hin zu 10⁴ - 10⁵ cd/m² im Außenbereich. Derartige Helligkeitsunterschiede sind simultan mit herkömmlichen Kameras und im übrigen auch mit dem menschlichen Auge nicht sichtbar. Die Helligkeitsanpassung erfolgt immer nur in dem jeweils betrachteten Bereich.

In der Figur 6 ist in Gestalt eines Diagramms dargestellt, wir das Vorverarbeitungsmodul der Kamera 1 aufgrund seiner Mustererkennungsfuktionen die einzelnen Bereiche voneinander abgrenzt und mit unterschiedlichen Funktionen in Helligkeitsstufen der Kameraabbildung umsetzt. Auf der x-Achse ist die Leuchtdichte in cd/m² über insgesamt 9 Dekaden aufgezeichnet, so wie sie im realen Bild der Figur 5 auftritt. Die y-Achse zeigt die 256 relativen Einheiten der Helligkeitsinformation, die von der Kamera bzw. deren Vorverarbeitungsmodul der Bilddarstellung zugewiesen wird. 256 Einheiten entsprechen einer Helligkeitsmodulation von 8 Bit.

Eine erste Leuchtdichtekurve L32 zeigt den Bereich der Leuchtdichte des Türschlosses 32, abgebildet auf die 256 relativen Helligkeitsstufen der Kameraabbildung. Entsprechende Helligkeitskurven L33 für das Türblatt 33, L34 für die Spinne 34 und L35 für den Außenbereich 35 sind ebenfalls dargestellt.

Das Vorverarbeitungsmodul erkennt in der eingehenden Abbildung unterschiedliche, konturenscharf voneinander abgegrenzte Bereiche mit den vier unterschiedlichen Helligkeitsbereichen. Diese Bereiche werden rechnerisch voneinander getrennt und jeweils mit optimaler Auflösung auf die 256 Helligkeitsstufen der Kameraabbildung transponiert. Im Ergebnis zeigt sich dem Betrachter der Szene gemäß Figur 5 ein Bild, bei dem die Bildbereiche 32, 33, 34 und 35 in der Darstellung gleich hell und mit entsprechender Strukturierüng in den verschiedenen Helligkeitsstufen dargestellt werden. Eine solche Darstellung kann ungewohnt sein, sie bietet aber einen Detailreichtum in verschiedenen Regionen, der weder mit dem menschlichen Auge noch mit herkömmlichen Kamerasystemen simultan darstellbar ist.

Die Abbildung gemäß Figur 5 zeigt auch Objekte unterschiedlicher Distanz. So sind die Objekte 32, 33 und 34 beispielsweise auf einer Entfernung von 2 m vom Betrachter angeordnet, während die dem Außenbereich 35 zugeordnete Palme 36 in einer Entfernung von 40 m stehen kann. Mit herkömmlichen Kameraobjektiven ist es in der Regel nicht möglich, beide Objekte 34 und 35 gleichzeitig scharf zu stellen. Die verfügbaren Tiefenschärfenbereiche reichen hierfür in der Regel nicht aus.

Der lernfähige sensomotorische Encoder kann gemeinsam mit dem Vorverarbeitungsmodul zunächst den entfernt liegenden Bereich 35 scharf stellen und dort konturenscharf abgegrenzte Regionen (Palme) erkennen und speichern. Sodann kann ein zweiter Entfernungsbereich gewählt werden, bei dem die Spinne 34 scharf eingestellt wird, während der Bereich 35 unscharf wird. Das Vorverarbeitungsmodul kann diesen Umstand erkennen und anstelle des unscharf gewordenen Bereichs 35 den zuvor ermittelten und gespeicherten scharfen Bildinhalt rechnerisch in das auf kurze Entfernung fokussierte Bild einfügen. Diese Abfolge kann in einer Art Fokusscanning zyklisch wiederholt werden, so daß ständig aus verschiedenen Fokussierungsentfernungen scharfe Bereiche ermittelt, gespeichert und zu einem insgesamt scharfen Bild zusammengefügt werden. Die Tiefenschärfe, die auf diese Art und Weise virtuell erreichbar ist, übersteigt diejenige eines normalen optischen Systems um ein Vielfaches. Bei ausreichender Wiederholfrequenz des Verfahrensablaufs ist für den Benutzer das erzeugte Bild von einem realen Bild nur anhand der besonders großen Tiefenschärfe zu unterscheiden.

Erfindungsgemäß wird ein Encoder vorgeschlagen, der diverse Funktionen durch neuronale Netze im Dialog mit dem Implantatträger optimiert, bei dem verschiedene Funktionsmoden angewählt sowie die Positionen erfaßter Objekte wahrgenommen werden können und der vor Hindernissen warnt, die technische Erkennung von Mustern meldet sowie die Zahl der selektiv erreichbaren Stimulationsorte funktionell erhöht und die neuronale Aktivität einzelner Neuronen überwacht. Die implantierte Struktur kann unter Verwendung geeigneter sensorischer und aktorischer Komponenten sowie eines lernfähigen Kontrollsystems teilweise autonom sensomotorisch agieren.

Vorteilhafte Ausgestaltungen und Weiterbildungen der Erfindung sind den Unteransprüchen entnehmbar.

Der lernfähige Encoder zeichnet sich gegenüber den bekannten Sehprothesensystemen durch eine Reihe von wesentlichen Vorteilen aus. Erstmals wird hier ein Encoder vorgeschlagen, der bereits von normalsichtigen Personen vortrainiert und anschließend vom Implantatträger dem Funktionsbedarf individuell angepaßt werden kann. Erstmals wird hier ein Encoder offenbart, der sowohl Augenbewegungsfunktionen leisten als auch unerwünschte Augenbewegungen kompensieren kann. Ferner wird erstmals ein Encoder offenbart, der die Zahl der selektiv erreichbaren Stimulationsorte funktionell erhöht und sich auch später an neue Stimulationsbedingungen anpassen kann. Ferner wird erstmals ein Encoder offenbart, der bidirektional funktioniert, also neben der Stimulationsfunktion auch eine Überwachung und Auswertung der neuronalen Aktivität der zu stimulierenden Neuronen gestattet. Entsprechende Vorteile ergeben sich bei Einsatz des lernfähigen Encoders gegenüber den bisher entwickelten Hörprothesensystemen.

Die Anpassungsfähigkeit der Abbildungsfunktionen der einzeln justierbaren spatiotemporalen Filter des Encoders mit rezeptiven Feldeigenschaften (RF-Filter) im gesamten neurophysiologisch relevanten Funktionsraum wird im Verband mit neuronalen Netzen oder anderen Parametereinstellverfahren beim Einsatz für Seh- oder Hörprothesen sichergestellt.

Die individuell im wahrnehmungsbasierten Dialog festzulegen-den Abbildungsfunktionen der einzelnen RF-Filter werden den vom Sehsystem erwarteten rezeptiven Feldeigenschaften hinreichend ähnlich, passen sich also der Funktion des aus Hintereinanderschaltung von Encoder und angekoppeltem zentralem Sehsystem bestehenden visuellen Systems an. Dies bedeutet einerseits, daß der durch RF-Filter bereitgestellte, spatiotemporale Funktionsraum den neurophysiologisch relevanten Funktionsraum einschließt und andererseits, daß die RF-Filter durch Parameterverstellung mit Hilfe eines neuronalen Netzes eine kontinuierliche Bewegung im Funktionsraum mit geeigneten Einstellverfahren gestatten. Entsprechendes gilt bei Einsatz des Encoders in Hörprothesen.

Bereits im Einsatz mit normalsichtigen Personen wird eine sinnvolle Voreinstellung der RF-Filter in Anlehnung an entsprechende neurophysiologische Daten zur Funktion des Sehsystems bzw. Hörsystems von Primaten vorgenommen. Das Dialogverfahren wird dazu mit den zugehörigen Komponenten unter realistischen Bedingungen durch Simulation des Wahrnehmungsprozesses erprobt. Entsprechendes gilt bei Einsatz des Encoders in Hörprothesen.

Die den einzelnen Mikrokontakten zugeordneten RF-Filter werden individuell im Dialog zwischen Encoder und Implantatträger auf optimale visuelle bzw. auditorische Wahrnehmungsqualität abgestimmt.

Im Unterschied zu einem Encoder mit starrer Vorverarbeitung, also ohne individuelle Verstellmöglichkeit, werden hier auf der Basis des einzig relevanten Kriteriums, nämlich der erzielten Sehwahrnehmung oder Hörwahrnehmung, die einzelnen RF-Filter als separate Encoderkanäle justiert. Spätere Funktionsänderungen der Sehprothese, z.B. infolge der Verlagerung von Mikrokontakten, oder von Änderungen des Funktionsanteiles. des zentralen Sehsystems am gesamten Wahrnehmungsprozeß können durch entsprechende Anpassungen der RF-Filterfunktion ausgeglichen werden. Ein Vorteil der Abstimmung der RF-Filter Funktion im Dialog mit dem Implantatträger besteht in der Berücksichtigung von Funktionsaspekten, die nur der jeweilige Implantatträger und nur in impliziter Form durch subjektive Bewertung seiner Sehwahrnehmung und deren Verwendung für die Encoderjustierung in den Optimierungsprozeß einbringen kann. Entsprechendes gilt für eine Hörprothese.

Die asynchronen Impulsfolgen der einzelnen RF-Filterausgänge der zunächst funktionell getrennten Encoderkanäle werden als Stimulationssignale für selektive Stimulationsorte im Dialog mit dem Implantatträger unter Berücksichtigung der registrierten neuronalen Impulse aufeinander abgestimmt.

Weil die zeitliche Kopplung bzw. Synchronisation der neuronalen Impulse mehrerer Neuronen zur neurobiologischen Signalcodierung in sensorischen Systemen mitverwendet wird, bringt diese hier technisch (auch durch Auswertung der registrierten neuronalen Aktivität zu stimulierender Neuronen) realisierte, zeitliche Kopplung den Vorteil einer Verbesserung der visuellen Wahrnehmungsqualität.

Die Zahl der selektiv erreichbaren Stimulationsorte und deren Trennschärfe bzw. Übersprechdämpfung bei einer festen Zahl von implantierten Mikrokontakten wird funktionell erhöht.

Bei einer gegebenen relativ geringen Zahl implantierter und dauerhaft funktionstüchtiger Mikrokontakte, deren Position relativ zu den Neuronen nicht modifiziert werden kann, ist es von großem Vorteil, funktionell, also durch Erzeugung geeigneter Signale, die Zahl der selektiv erreichbaren Stimulationsorte bzw. Neuronen und damit gleichzeitig die Zahl der separat verfügbaren Encoderkanäle bei einer hinreichenden Reserve an RF-Filtern zu erhöhen. Dies bewirkt eine Verbesserung der visuellen Wahrnehmungsqualität.

Die Detektion der Augen- und Kopfbewegungen hat den Vorteil der Bestimmung der aktuellen Position von visuellen Objekten im Raum. Ferner besteht ein Vorteil darin, unerwünschte reale Augenbewegungen durch entsprechend simulierte Augenbewegungen zu kompensieren und ferner Sehwahrnehmungskonflikte, wie z.B. Scheinbewegungen, oder Schwindel zu unterdrücken.

Die Erzeugung der einzelnen Bewegungsfunktionen als separat oder kombiniert wählbare Programme hat den Vorteil, daß der Implantatträger die Programme je nach dem Nutzen für die visuelle Wahrnehmungsqualität selbst wählen kann, statt einer automatischen Funktion unterworfen zu sein. Dennoch ist eine Entscheidung zwischen Automatik und Wahlbetrieb möglich.

Es ist für den Implantatträger von sehr großer Bedeutung, die aktuelle Position eines wahrgenommenen visuellen oder auditorischen Objektes wahrnehmen zu können, um seine Orientierung im Raum und ggf. Handlungen danach richten zu können. Ferner ist es von großem Vorteil, daß der Implantatträger vor Hindernissen bzw. Gefahren automatisch gewarnt wird und die technische Erkennung von Mustern oder Objekten zur Unterstützung der Orientierung im Raum gemeldet wird.

Mit dem Encoder wird eine direkte Verbindung zu einem Teil des Nervensystems hergestellt, welches bereits spontan aktiv ist. Es werden also neuronale Impulse von einzelnen Neuronen ohne technische Stimulation generiert. Zur optimalen Anpassung der Stimulationspulsfolgen an die jeweilige Spontanaktivität, zur genauen Bestimmung der Stimulationsparameter für eine sichere und gleichzeitig noch biologisch verträgliche Umsetzung von Stimulationspulsen in neuronale Impulse sowie zur besseren Optimierung der zeitlichen Abstimmung und Synchronisation der neuronalen Aktivität mehrerer Neuronen ist die Überwachung der neuronalen Aktivität einzelner zu stimulierender Neuronen von großem Vorteil.

Mit der technischen Anpassung des Arbeitsbereiches ist es möglich, im physiologisch helladaptierten oder dunkeladaptierten Leuchtdichtebereich die Funktion an die Bildmuster oder Schallmuster anzupassen, die spatiotemporalen Filterparameter entsprechend zu variieren, oder einen Arbeitsbereich technisch zusammenzustellen, der z.B. aus um Dekaden voneinander entfernten Teilbereichen des großen Photosensorfunktionsbereiches besteht.

Vorverarbeitung eintreffender Bildmuster insbesondere bezüglich mit schneller Wahl- und Änderungsmöglichkeit der jeweiligen Vorverarbeitungsfunktionen wird erfindungsgemäß nach dem Vorschlag gemäß Anspruch 7 ermöglicht.

Mit dem vorgeschalteten Vorverarbeitungsmodul kann die Funktion des aus einer nur begrenzten Zahl von RF-Filtern bestehenden Encoders durch wesentliche Vereinfachung des Bildmusters oder Schallmusters erleichtert und entsprechend die Wahrnehmungsqualität verbessert werden.

## Patentansprüche

1. Lernfähiger, sensomotorischer Encoder (1) für eine Sehprothese oder Hörprothese, mit einer zentralen Kontrolleinheit für Signalverarbeitungsfunktionen, überwachungsfunktionen, Steuerfunktionen und externe Eingriffsfunktionen, sowie mit einer Gruppe von adaptiven spatiotemporalen Filtern zur Umsetzung von Sensorsignalen in Stimulationspulsfolgen, **dadurch ge- kennzeichnet** , daß die Sehprothese oder Hörprothese eine bidirektionale Schnittstelle zur Kopplung des Encoders (1) mit einer implantierbaren Mikrostruktur (2,6) zur Stimulation von Nerven- oder Gliagewebe einerseits sowie zur Funktionsüberwachung von Hirnfunktionen andererseits vorgesehen ist.

2. Encoder nach Anspruch 1, **dadurch ge- kennzeichnet**, daß die Kontrolleinheit in einem wahrnehmungsbasierten Dialogverfahren einstellbare spatiotemporale Filter mit rezeptiven Feldeigenschaften (RF-Filter) umfaßt.

3. Encoder nach einem der vorhergehenden Ansprüche, **da- durch gekennzeichnet** , daß ein simuliertes Augenbewegungssystem mit Kopf- und Augenbewegungsdetektoren (K,A) vorgesehen ist.

4. Encoder nach einem der vorhergehenden Ansprüche, **da- durch gekennzeichnet** , daß den RF-Filtern steuerbare Zeitverzögerungsglieder für die relative zeitliche Abfolge der erzeugten Impulsfolgen (S₁,8₂,S₃) zugeordnet sind.

5. Encoder nach einem der vorhergehenden Ansprüche, **da- durch gekennzeichnet** , daß ein Monitor vorgesehen ist, der die von den RF-Filtern erzeugte Abbildungsfunktion zumindest näherungsweise invertiert und optisch oder akustisch darstellt.

6. Encoder nach einem der vorhergehenden Ansprüche, **da- durch gekennzeichnet** , daß implantatseitig eine Vorrichtung zur von der zentralen Kontrolleinheit gesteuerten Applikation eines Wirkstoffs vorgesehen ist und daß implantatseitig die neuronale Aktivität von Neuronen registriert und an ein Überwachungssystem der zentralen Kontrolleinheit übergeben wird.

7. Encoder nach einem der vorhergehenden Ansprüche, **da- durch gekennzeichnet** , daß ein lernfähiges Vorverarbeitungsmodul in der zentralen Kontrolleinheit des Encoders (1) zur Vereinfachung von Bildmustern oder Schallmustern vorgesehen ist.

8. Verfahren zum Betrieb eines Encoder nach einem der vorhergehenden Ansprüche, **dadurch gekenn- zeichnet** , daß
- zur individuellen Einstellung der Signalverarbeitungsfunktionen in einem Dialogverfahren eine wenigstens einkanalige Bewertungseingabeeinheit (3) mit einem subjektiven Bewertungsvektor gespeist wird, wobei der Bewertungsvektor die Ähnlichkeit des jeweils wahrgenommenen Bildmusters - oder Schallmusters - mit einem Sollmuster repräsentiert,
- daß der Bewertungsvektor in dem Dialogmodul (3) an ein Parametereinstellungssystem zur Erzeugung geeigneter Folgen von Parametervektoren, insbesondere an ein neuronales Netz mit nicht-überwachter Lernregel, übergeben wird,
- und daß das Dialogmodul (3) an einem mehrkanaligen Ausgang einen Parametervektor für die jeweils einzustellenden Signalverarbeitungsfunktionen erzeugt.

9. Verfahren nach Anspruch 8, **dadurch gekenn- zeichnet**, daß die Signalverarbeitungsfunktionen von RF-Filtern gebildet werden.

10. Verfahren nach einem der vorhergehenden Ansprüche, **dadurch gekennzeichnet , daß** das Dialogmodul (3) in einem Entscheidungssystem geeignete Mustersequenzen für die Lernphase zur einzelnen oder gruppenweisen Optimierung der RF-Filter festlegt.

11. Verfahren nach einem der vorhergehenden Ansprüche, **dadurch gekennzeichnet , daß** intern gespeicherte Sequenzen von Parametervektoren zur Festlegung von typischen RF-Filterfunktionen erzeugt werden.

12. Verfahren nach einem der vorhergehenden Ansprüche, **dadurch gekennzeichnet , daß** die RF-Filter einen spatiotemporalen Funktionsraum haben, der den Funktionsraum der rezeptiven Feldeigenschaften visueller oder auditorischer Neuronen am jeweiligen Implantationsort einschließt.

13. Verfahren nach einem der vorhergehenden Ansprüche, **dadurch gekennzeichnet , daß** ein von den RF-Filtern erzeugtes Signal (S₁,S₂,S₃) zur Auslösung einer Ist-Wahrnehmung an die implantierbare Mikrostruktur (2,6) und simultan das dazugehörige Sollmuster an eine zweite Ausgabeeinheit, die einem anderen menschlichen Sinnesorgan zugänglich ist, abgegeben wird.

14. Verfahren nach einem der vorhergehenden Ansprüche, **dadurch gekennzeichnet , daß** zur funktionellen Erhöhung der Zahl und Trennschärfe der selektiv erreichbaren Stimulationsorte (F,F') bei einer gegebenen Zahl von stationär implantierten Mikrokontakten (2,6) die Impulssignale (S₁,S₂,S₃) von jeweils einem RF-Filter an mehrere, örtlich benachbarte Mikrokontakte (16,17,18) geleitet werden.

15. Verfahren nach einem der vorhergehenden Ansprüche, **dadurch gekennzeichnet , daß** die selektiven Stimulationsorte (F,F') durch geeignete Variation der superponierten Impulssignale (S₁,S₂,S₃) zur örtlichen Verlagerung des Stimulationsfocus (F,F') schnell gewechselt werden können.

16. Verfahren nach einem der vorhergehenden Ansprüche, **dadurch gekennzeichnet , daß** die Variation der Stimulationssignale (S₁,S₂,S₃) zur Verlagerung des Stimulationsfocus (F,F') im wahrnehmungsbasierten Dialog mit dem Implantatträger über ein neuronales Netz oder andere Optimierungsalgorithmen in der zentralen Kontrolleinheit zur Bestimmung möglichst vieler, zu selektiver und trennscharfer neuronaler Erregung führender Stimulationsorte erfolgt.

17. Verfahren nach einem der vorhergehenden Ansprüche, **dadurch gekennzeichnet, daß** durch Vergleich registrierter neuronaler Impulse mit den Stimulationssignalen (S₁,S₂,S₃) die aus spontan auftretenden neuronalen Impulsen und durch Stimulation erzeugten zusätzlichen Impulsen zusammengesetzte neuronale Erregung bezüglich der Selektivität und der Bioverträglichkeit verbessert wird.

18. Verfahren nach einem der vorhergehenden Ansprüche, **dadurch gekennzeichnet, daß** zur Simulation von Augenbewegungen Kopfbewegungen und unerwünschte reale Augenbewegungen detektiert werden, daß Augenbewegungen mittels elektronischer Bildmusterverschiebung und/oder optischer Variation der Blickrichtung und/oder Bewegung der Photosensoren simuliert werden, daß unter Verwendung der detektierten realen Kopf- und Augenbewegungssignale sowie der Simulation der Augenbewegungen mittels einer Bewegungssteuerung oder -regelung schnelle und langsame Augenbewegungen für Aufgaben der Mustererkennung , Verfolgung bewegter Objekte und zum schnellen Umherblicken erzeugt werden.

19. Verfahren nach einem der vorhergehenden Ansprüche, **dadurch gekennzeichnet , daß** Kopfbewegungen und reale Augenbewegungen detektiert werden und daß unter Verwendung der detektierten Kopf- und Augenbewegungen mit Hilfe einer Bewegungssteuerung oder -regelung schnelle und langsame simulierte Augenbewegungen erzeugt werden, um unerwünschte reale Augenbewegungen oder Scheinbewegungswahrnehmungen zu kompensieren.

20. Verfahren nach einem der vorhergehenden Ansprüche, **dadurch gekennzeichnet , daß** zur Lagestabilisierung der Bildmuster bei natürlichen Kopf-Rumpfbewegungen kompensatorische Augenbewegungen in Anlehnung an den vestibulo-okulären Reflex erzeugt werden.

21. Verfahren nach einem der vorhergehenden Ansprüche, **dadurch gekennzeichnet, daß** zur technischen Anpassung des Leuchtdichtearbeitsbereiches aus dem über mehrere Leuchtdichtedekaden reichenden Funktionsbereich des Photosensorarrays (4) ein Arbeitsbereich für den Encoder (1) bezüglich Größe und Adaptationsleuchtdichte gewählt werden kann, und daß dieser ausgewählte Arbeitsbereich aus nichtlinear bewerteten Ausschnitten des Funktionsbereichs zusammengesetzt werden kann.

22. Verfahren nach einem der vorhergehenden Ansprüche, **dadurch gekennzeichnet , daß** durch ein geeignetes Abbildungsystem visuelle Szenen (32,33,34,35) mit örtlichen Teilbereichen sehr unterschiedlicher Leuchtdichte (L32,L33,L34,L35) in den jeweiligen, gemeinsamen Leuchtdichtearbeitsbereich des Encoders transponiert werden können und damit beispielsweise die visuelle Wahrnehmung invariant gegenüber einer Veränderung der mittleren Szenenleuchtdichte gemacht wird.

23. Verfahren nach einem der vorhergehenden Ansprüche, **dadurch gekennzeichnet , daß** die RF-Filterfunktionen zur Simulation der Wahrnehmung im hell- oder dunkel adaptierten Bereich bei Bedarf mit dem Leuchtdichte-Arbeitsbereich des Encoders verstellt werden.

24. Verfahren nach einem der vorhergehenden Ansprüche, **dadurch gekennzeichnet , daß** sowohl die Leuchtdichte-Arbeitsbereichswahl als auch die zugehörige RF-Filterverstellung insbesondere im wahrnehmungsbasierten Dialog automatisch während simulierter Augenbewegungen, oder während der Mustererkennung schnell verändert werden können.

25. Verfahren nach einem der vorhergehenden Ansprüche, **dadurch gekennzeichnet , daß** in einem Ablauf durch Variation der Akkommodation zunächst erste Bereiche (35,36) des Blickfeldes scharf eingestellt werden, diese Bereiche (35,36) gespeichert werden, sodann zweite Bereiche (32,33,34) des Blickfeldes als Bild scharf eingestellt werden, wobei die ersten Bereiche (35,36) unscharf werden, und daß die ersten gespeicherten Bereiche (35,36) anstelle der ersten unscharf gewordenen Bereiche (35,36) in das Bild eingeblendet werden.

26. Verfahren nach einem der vorhergehenden Ansprüche, **dadurch gekennzeichnet , daß** der Ablauf sich zyklisch wiederholt.

27. Verfahren nach einem der vorhergehenden Ansprüche, **dadurch gekennzeichnet , daß** die vom Encoder (1) unter Auswertung von Bildmuster-, Augen-und Kopfbewegungen ermittelte Position eines visuell erfaßten Objektes im Raum, oder daß die unter Auswertung von Schallmusterbewegungen ermittelte Position eines auditorisch erfaßten Objektes im Raum mit einem geeigneten, mitgeführten Signalgeber (H) an ein geeignetes Sinnesorgan gemeldet wird.

28. Verfahren nach einem der vorhergehenden Ansprüche, **dadurch gekennzeichnet , daß** der Encoder durch interne Mustererkennungsprogramme, insbesondere in Verbindung mit automatisch ablaufenden, simulierten Augenbewegungen, den Implantatträger vor Hindernissen oder Gefahren warnt sowie Art und Position technisch erkannter Muster oder Objekte meldet.
